# EUROPEAN PATENT SPECIFICATION

(11) **EP 3 768 229 B1**
(45) Date of publication and mention of the grant of the patent: **11.12.2024**
(21) Application number: 19711622.1
(22) Date of filing: 25.03.2019
(51) Int. Cl.: A61K 8/368, A61K 8/39, A61Q 19/00, A61K 8/92, A61K 31/192, A61P 17/16, A61P 17/06

(54) **SKIN CONDITION IMPROVING AGENTS**
MITTEL ZUR VERBESSERUNG DES HAUTZUSTANDS
AGENTS AMÉLIORANT L'ÉTAT DE LA PEAU

(30) Priority: 23.03.2018 US 201862647158 P; 04.04.2018 WO PCT/EP2018/058632
(43) Date of publication of application: 27.01.2021
(73) Proprietor: Symrise AG, 37603 Holzminden Niedersachsen (DE)
(72) Inventor: SCHMAUS, Gerhard, 37671 Höxter-Bosseborn (DE); LANGE, Sabine, 37603 Holzminden (DE); SLAVASHEVICH, Paul, BRONX,, New York 10471 (US)
(74) Representative: Eisenführ Speiser
(86) International application number: PCT/EP2019/057387
(87) International publication number: WO 2019/180266

(56) References cited:
- EP-A1- 3 108 941
- US-A1- 2010 215 775
- US-A1- 2016 015 031
- US-A1- 2016 279 074
- US-B2- 8 476 318
- US-B2- 8 911 795
- GERHARD SCHMAUS ET AL: "Use of anthranilic acid amides as antioxidants in food, functional food, cosmetic and pharmaceutical compositions", RESEARCH DISCLOSURE, KENNETH MASON PUBLICATIONS, HAMPSHIRE, UK, GB, vol. 506, no. 3, 1 June 2006 (2006-06-01), XP007136279, ISSN: 0374-4353
- GERHARD SCHMAUS AND RAVI PILLAI ET AL: "Cosmetic and pharmaceutical compositions containing anthranilic acid amides or derivatives thereof and further anti-irritant and/or anti-itch ingredients", RESEARCH DISCLOSURE, KENNETH MASON PUBLICATIONS, HAMPSHIRE, UK, GB, vol. 505, no. 11, 1 May 2006 (2006-05-01), XP007136151, ISSN: 0374-4353
- DATABASE GNPD [online] MINTEL; 17 January 2018 (2018-01-17), ANONYMOUS: "Cream in Oil", XP055523058, retrieved from www.gnpd.com Database accession no. 5383393
- DATABASE GNPD [online] MINTEL; 15 February 2018 (2018-02-15), ANONYMOUS: "Skincare Routine: Dry Skin", XP055523056, retrieved from www.gnpd.com Database accession no. 5449531

## Description

The present invention relates to a composition comprising Avenanthramide(s) and / or polyalkylene glycol derivative(s) as well as oil bodies. One aspect of the present invention relates to such a composition for use in a therapeutic method for improving or preventing one or more undesired skin conditions. The present invention further relates to a cosmetic and / or dermatological preparation comprising such a composition.

Several skin diseases but also skin aging is associated with thinner, dry skin showing reinforced and undesired clinical symptoms like excoriation, lichenification and scaling.

Excoriation may typically be caused by excoriation disorder, with the onset of acne in adolescence or other skin conditions such as keratosis pilaris, psoriasis and eczema. Excoriation describes several skin lesions or abrasions caused by e.g. excessive grooming of the skin.

Lichenification describes a skin condition including epidermal thickening, typically characterized by visible and palpable thickening of the skin. Frequently, accentuated skin markings and / or an exaggeration of normal skin lines appear.

Scaling describes an abnormal shedding or accumulation of the upper layer of the skin (stratum corneum). Scaling is often associated with dry skin disorders.

The skin may tend to scaling e.g. because it is too dry. A physiological mechanism to prevent scaling by moisturizing the skin is the production of grease. However, with increasing age, use of e.g. soaps, shampoos, perfumes or disinfectants, contact with hot water and dry air conditions, e.g. caused by indoor heating, the skin may become dry, rough and start scaling. In general, substances causing de-fattening or degreasing and thus drying of the skin seem to promote the generation of undesired clinical symptoms such as e.g. excoriation, lichenification and dry skin (e.g. scaling).

WO 2016/207084A1 describes the use of polyethylene glycol esters and / or polyethylene glycol ethers in the treatment of itchy conditions of skin and scalp.

US 2010/150854A1 describes cosmetic and / or dermatological preparations containing polyethylene glycol esters and / or polyethylene glycol ethers for moisturizing the skin and to delay or prevent drying out of the skin.

DE 102 54 872 A1 relates to the use of Anthranilic acid amides as a cosmetic preparation for inhibiting the substance P induced release of Histamine. A reduction of Histamine release is reported to improve skin itching, pain and reddening of the skin.

US 2016/0015031 A1 refers to antimicrobial agents for personal care, detergent and nutrition products, wherein Dihydroavenanthramide may be present as anti-reddening or anti-itching agent.

Schmaus et al.: "Use of anthranilic acid amides as antioxidants in food, functional food, cosmetic and pharmaceutical compositions", RESEARCH DISCLOSURE, KENNETH MASON PUBLICATIONS, HAMPSHIRE, UK, GB, vol. 506, no. 3, 1 June 2006 (2006-06-01), as well as Schmaus and Pillai et al: "Cosmetic and pharmaceutical compositions containing anthranilic acid amides or derivatives thereof and further anti-irritant and/or anti-itch ingredients", RESEARCH DISCLOSURE, KENNETH MASON PUBLICATIONS, HAMPSHIRE, UK, GB, vol. 505, no. 11, 1 May 2006 (2006-05-01) describe the use of anthranilic acid amides as antioxidants in food, functional food, cosmetic and pharmaceutical compositions and describe compositions comprising Dihydroavenanthramide and PEG derivatives.

US 8,911,795 B2 describes compositions comprising Dihydroavenanthramide D and climbazole, useful for treating skin and/or hair, wherein the compositions may comprise a PEG derivative.

US 2010/215775 A1 describes the use of C10-C14-alkane-1,2-diols in the preparation of a composition for the prophylaxis and/or treatment of Malassezia-induced dandruff formation, wherein the compositions presented in the Examples may further comprise Dihydroavenanthramide D and/or PEG derivatives.

However, further substances and cosmetic and / or dermatological preparations with increased effectiveness in improving or preventing undesired skin conditions are sought.

In the search for suitable agents it has to be considered that the substances used should be toxicologically acceptable, well tolerated by the skin and stable (in particular in conventional cosmetic and / or dermatological formulations) and they should preferably have the lowest possible intrinsic odour and the lowest possible intrinsic colour and be inexpensive to prepare.

The object of the present invention was therefore to develop a stable, cost-effective product with skin condition improving properties. Such skin conditions preferably comprise or consist of excoriation, lichenification and scaling associated with dry skin.

The object described above is solved by a composition according to the invention, comprising
a) one, two, three or more Avenanthramide(s) of formula (I) 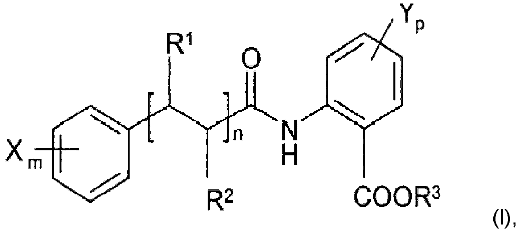 wherein
   m = 0, 1,2 or 3,
   p = 0, 1 or 2,
   n = 0, 1 or 2,
   providing that if n is 1 or 2, the sum (p + m) is > 0,
   wherein if n is 1 or 2, both R¹ and R² are H or together form a further chemical bond,
   wherein if m is 1, 2 or 3, each X is independent of the other(s) OH, O-Alkyl or O-Acyl,
   wherein if p is 1 or 2, each Y is independent of the other(s) OH, O-Alkyl or O-Acyl,
   providing that if (p + m) > 0, X or Y is at least once selected from the group consisting of OH and O-Acyl,
   wherein R³ = H, N or Alkyl,
   wherein the or an Avenanthramide of formula (I) is Dihydroavenanthramide D,
   and two, three or more polyalkylene glycol derivative(s) according to formula (II): R¹(OCH₂CHR²)ₙOR³,
   in which
   R¹ stands for hydrogen or a linear or branched alkyl group with 1 to 4 carbon atoms,
   R² stands for hydrogen or methyl,
   n represents an integer of from 3 to 20
   R³ stands for a linear or branched alkyl or alkenyl group with 6 to 18 carbon atoms and 0, 1, 2 or 3 double bonds or a -COR⁴ acyl group, and
   R⁴ stands for hydrogen, a linear or branched alkyl or alkenyl group having 5 to 17 carbon atoms, or an alkali metal, an alkaline earth metal or NH₃,
   wherein the or two polyalkylene glycol derivatives according to formula (II) are Polyoxyethylene (9) tridecyl ether and PEG-5 Ethylhexanoate
   and optionally
b) oil bodies,
   wherein
   the ratio of the total amount of the Avenanthramide(s) to the total amount of the polyalkylene glycol derivative(s) in the composition is in the range of from 1 : 25 to 1:100 based on weight.

One, two, three or more further Avenanthramide(s) of formula (I) in the composition according to the invention is / are or, respectively, comprise preferably one or two Avenanthramide(s) selected from the group consisting of Avenanthramides A, B, C, D and Dihydroavenanthramides A, B, C.

One, two, three or more further polyalkylene glycol derivative(s) of formula (II) in the composition according to the invention is / are or, respectively, comprise preferably one, two, three or more polyalkylene glycol derivative(s) according to formula (Ila)

H(OCH₂CH₂)ₙOR³ (Ila)

in which n represents an integer of from 7 to 12 and R³ stands for a linear or branched alkyl group with 8 to 15 carbon atoms,
and / or is / are preferably one, two, three or more polyalkylene glycol derivative according to formula (IIb)

   H(OCH₂CH₂)ₙO-COR⁴ (IIb)
in which n represents an integer of from 3 to 7 and R⁴ stands for a linear or branched alkyl group with 7 to 11 carbon atoms.

One, two, three or more further polyalkylene glycol derivative(s) of formula (II) and / or (Ila) and / or (IIb) in the composition according to the invention is / are or, respectively, comprise preferably one, two, three or more polyethylene glycol (PEG) ether(s) of formula (Ila) and / or one, two, three or more polyethylene glycol (PEG) ester(s) of formula (IIb).

Polyethylene glycol ethers according to formula (II) and / or (Ila) that can be additionally used in a composition are for example PEG-7 Octylether, PEG-7 Nonylether, PEG-7 Decylether, PEG-7 Undecylether, PEG-7 Dodecylether, PEG-7 Tridecylether, PEG-7 Tetradecylether, PEG-8 Octylether, PEG-8 Nonylether, PEG-8 Decylether, PEG-8 Undecylether, PEG-8 Dodecylether, PEG-8 Tridecylether, PEG-8 Tetradecylether, PEG-9 Octylether, PEG-9 Nonylether, PEG-9 Decylether, PEG-9 Undecylether, PEG-9 Dodecylether, PEG-9 Tetradecylether, PEG-10 Octylether, PEG-10 Nonylether, PEG-10 Decylether, PEG-10 Undecylether, PEG-10 Dodecylether, PEG-10 Tridecylether, PEG-10 Tetradecylether, PEG-11 Octylether, PEG-11 Nonylether, PEG-11 Decylether, PEG-11 Undecylether, PEG-11 Dodecylether, PEG-11 Tridecylether, PEG-11 Tetradecylether, PEG-12 Octylether, PEG-12 Nonylether, PEG-12 Decylether, PEG-12 Undecylether, PEG-12 Dodecylether, PEG-12 Tridecylether, PEG-12 Tetradecylether.

Preferably, the further PEG ether or one of the further PEG ethers in the composition according to the invention is Polyoxyethylene lauryl ether (Laureth 9).

Polyethylene glycol esters that can be additionally used in a composition according to formula (II) and / or (IIb) are for example PEG-3 Octanoate, PEG-3 Nonanoate, PEG-3 Isononanoate, PEG-3 Decanoate, PEG-3 Undecanoate, PEG-3 Dodecanoate, PEG-4 Octanoate, PEG-4 Nonanoate, PEG-4 Isononanoate, PEG-4 Decanoate, PEG-4 Undecanoate, PEG-4 Dodecanoate, PEG-5 3,5,5-Trimethylhexanoate, PEG-5 Octanoate, PEG-5 Nonanoate, PEG-5 Isononanoate, PEG-5 Decanoate, PEG-5 Undecanoate, PEG-5 Dodecanoate, PEG-6 Octanoate, PEG-6 Nonanoate, PEG-6 Isononanoate, PEG-6 Decanoate, PEG-6 Undecanoate, PEG-6 Dodecanoate, PEG-7 Octanoate, PEG-7 Nonanoate, PEG-7 Isononanoate, PEG-7 Decanoate, PEG-7 Undecanoate or PEG-7 Dodecanoate.

Preferably, the further PEG ester or one of the further PEG esters in the composition according to the invention is PEG-5 3,5,5-Trimethylhexanoate.

It is further preferred that the, or one further polyalkylene glycol derivative is PEG-5-3,5,5-Trimethylhexanoate.

Oil bodies that can be used in a composition according to the invention are for example Guerbet alcohols based on fatty alcohols having 6 to 18, preferably 8 to 10, carbon atoms, esters of linear C6-C22-fatty acids with linear or branched C6-C22-fatty alcohols or esters of branched C6-C13-carboxylic acids with linear or branched C6-C22-fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl, palmitate, stearyl stearate, stearyl isostearate, stearyl oleate, stearyl behenate, stearyl eru-cate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C6-C22-fatty acids with branched alcohols, in particular 2-ethylhexanol, esters of C18-C38-alkylhy-droxy carboxylic acids with linear or branched C6-C22-fatty alcohols, in particular Dioctyl Malate, esters of linear and / or branched fatty acids with polyhydric alcohols (such as, for example, propylene glycol, dimerdiol or trimertriol) and / or Guerbet alcohols, triglycerides based on C6-C10-fatty acids, liquid mono-/di-/triglyceride mixtures based on C6-C18-fatty acids, esters of C6-C22-fatty alcohols and / or Guerbet alcohols with aromatic carboxylic acids, in particular benzoic acid, esters of C2-C12-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C6-C22-fatty alcohol carbonates, such as, for example, Dicaprylyl Carbonate (Cetiol^{®} CC), Guerbet carbonates, based on fatty alcohols having 6 to 18, preferably 8 to 10, carbon atoms, esters of benzoic acid with linear and / or branched C6-C22-alcohols (e.g. Finsolv^{®} TN), linear or branched, symmetrical or asymmetrical dialkyl ethers having 6 to 22 carbon atoms per alkyl group, such as, for example, dicaprylyl ether (Cetiol^{®} OE), ring-opening products of epoxidized fatty acid esters with polyols, silicone oils (cyclomethicones, silicone methicone grades, etc.) and / or aliphatic or naphthenic hydrocarbons, such as, for example, squalane, squalene or dialkylcyclohexanes.

In case several different Avenanthramides are present in the composition, the weight ratio of the Avenanthramide with the highest weight proportion and the, one or all other(s) Avenanthramide(s) is preferably from 10 : 1 to 1 : 10, preferably to more than 1 : 1, more preferably from 5 : 1 to 1 : 5, preferably to more than 1 : 1, and particularly preferably from 2 : 1 to 1 : 2, preferably to more than 1 : 1.

In case several different polyalkylene glycol derivatives are present in the composition, the weight ratio of the polyalkylene glycol derivative with the highest weight proportion and the, one or all other(s) polyalkylene glycol derivative(s) is preferably from 10 : 1 to 1 : 10, preferably to more than 1 : 1, more preferably from 5 : 1 to 1 : 5, preferably to more than 1 : 1, and particularly preferably from 2 : 1 to 1 : 2, preferably to more than 1 : 1.

In case several different polyethylene glycol derivatives, several different polyethylene glycol ethers and / or several different polyethylene glycol esters are present in the composition, said ratios apply accordingly and are preferred as well.

In a further embodiment of the present invention, component a) of the composition according to the invention consists of one, two, three or more Avenanthramide(s) of formula (I) and of two, three or more polyalkylene glycol derivative(s) of formula (II), preferably of formula (Ila) and / or formula (IIb), wherein component a) at least comprises Dihydroavenanthramide D, Polyoxyethylene (9) tridecyl ether and PEG-5 Ethylhexanoate.

In another embodiment of the present invention, one or the, two, three or more further polyalkylene glycol derivative(s) of the composition according to the present invention is / are or, respectively, comprise (a) polyethylene glycol ester(s), preferably wherein the number of C-atoms of the polyethylene glycol part is 3 to 7 and / or the number of C-atoms of the ester part is 3 to 5 and / or (a) polyethylene glycol ether(s), preferably wherein the number of C-atoms of the polyethylene glycol part is 7 to 11 and / or the number of C-atoms of the ether part is 11 to 15.

The term "polyethylene glycol part" of an ester or ether is meant to be understood as the part of the polyethylene glycol ester or ether which originates from polyethylene glycol.

The term "ester part" is meant to be understood as the part of the polyethylene glycol ester which originates from the acid, which formed an ester bond with polyethylene glycol resulting in the respective polyethylene glycol ester.

The term "ether part" is meant to be understood as the part of the polyethylene glycol ether which originates from the compound, which formed an ether bond with polyethylene glycol resulting in the respective polyethylene glycol ether.

In one embodiment of the present invention, the total amount of component a) and / or b), if present, in the composition is sufficient to improve or prevent one or more undesired skin condition(s), wherein the undesired skin condition(s) is / are preferably one, two or more condition(s) selected from the group consisting of excoriation, lichenification and scaling associated with dry skin.

For the purposes of the present invention, "skin" is in particular the organ covering the outside of the human body and consisting of the epidermis, dermis and subcutis. Preferably, linings and / or coverings of internal organs, for example mucous membranes, periostea, vascular tissue and the retina are not "skin" for the purposes of the present invention.

The presence of polyalkylene glycol ester(s), particularly (a) polyethylene glycol ester(s) and (a) polyethylene glycol ether(s) in such a composition surprisingly showed a reduction in the clinical symptoms associated with dry skin such as scaling, excoriation and lichenification (see example 2).

Even more surprising, a combination of (an) Avenanthramide(s) and polyalkylene glycol ester(s), particularly (a) polyethylene glycol ester(s) and (a) polyethylene glycol ether(s) in such a composition shows reinforced reduction of the clinical symptoms associated with dry skin such as scaling, excoriation and lichenification (see example 3) although such effects hitherto have never been described for Avenanthramides.

In another embodiment of the present invention, the composition according to the invention can be used in a therapeutic method for improving or preventing one or more undesired skin condition(s), wherein the undesired skin condition(s) is / are preferably one, two or more condition(s) selected from the group consisting of excoriation, lichenification and scaling associated with dry skin.

A further aspect of the present invention relates to a cosmetic and / or dermatological preparation comprising a composition according to the invention, wherein such composition is present in a total amount sufficient to improve or prevent one or more undesired skin condition(s), wherein the undesired skin condition(s) is / are preferably one, two or more condition(s) selected from the group consisting of excoriation, lichenification and scaling associated with dry skin.

"Cosmetic" preparations for the purposes of the present invention are in particular preparations which are suitable for topical application to the skin of a human being, in particular to achieve a cosmetic effect. "Dermatological preparations" are preparations suitable for topical application to the skin of a human being to achieve a pharmaceutical effect, in particular to alleviate or cure a disease, in particular a skin disease.

It has also surprisingly been found that a composition according to the invention containing polyalkylene glycol derivative(s) has excellent solubilizing properties with regard to further, only moderately water-soluble, lipophilic substances or substance mixtures such as for example perfume oils, antidandruff agents, antiacne agents or substances regenerating the skin barrier. In particular, the very good solubility mediating properties of the composition according to the invention with regard to other active ingredients such as for example fatty oils, fatty acids, ceramides, pseudoceramides, sterols, phytosterols or hydrocarbons ideally suit them to be used in cosmetic and dermatological agents to improve or prevent one or more undesired skin conditions, in particular selected from the group consisting of excoriation, lichenification and scaling associated with dry skin.

In one embodiment of the present invention, the cosmetic and / or dermatological preparation is an oil-in-water (o/w) emulsion, preferably a two-phase system of an oil in water or a shampoo.

In another embodiment of the present invention, the total amount of polyalkylene glycol derivative(s) in the cosmetic and / or dermatological composition according to the invention, accounts for 0.1 to 5.0 wt.-%, preferably 0.5 to 2.5 wt.-%, of the preparation.

In a further embodiment of the present invention, the total amount of the Avenanthramide(s) in the cosmetic and / or dermatological composition according to the invention, accounts for 0.005 to 1.0 wt.-%, preferably 0.01 to 1.0 wt.-%, preferably 0.05 to 1.0 wt.-%, preferably 0.005 to 0.20 wt.-%, preferably 0.01 to 0.20 wt.-%, 0.02 to 0.20 wt.-%, of the preparation.

In another embodiment of the present invention, the cosmetic and / or dermatological preparation further comprises one or more moderately water-soluble active ingredients selected from the group consisting of odoriferous substances, perfume oils, aroma substances, aromas, fatty oils, fatty acids, waxes, ceramides, pseudoceramides, sterols, phytosterols, antiacne active ingredients, antidandruff active ingredients, antimicrobial active ingredients, preservatives, antiperspirants, antiirritants, pruritus-relieving active ingredients, cooling active ingredients, antioxidants, UV filters, antiaging active ingredients, skin-lightening and skin-tanning active ingredients, skin moisture regulators, osmolytes, insect repellents, enzyme inhibitors, odor absorbers, dyes.

In a further embodiment of the present invention, the cosmetic and / or dermatological preparation can be used in a therapeutic method for improving or preventing one or more undesired skin conditions, wherein the undesired skin condition(s) is / are preferably one, two or more conditions selected from the group consisting of excoriation, lichenification and scaling associated with dry skin.

The compounds as described herein are meant to be understood as the respective compounds, their stereoisomers and / or their respective salts.

Preferred embodiments and further aspects of the present invention emerge from the attached patent claims and the following examples, the examples not being intended to limit the scope of the present invention.

### Examples

### Example 1.1: Compositions according to the invention

| **Substance** | **Avenanthramide [wt.-%]** | **Polyalkylene glycol derivate(s) [wt.-%]** | **Oil bodies [wt.-%]** |
|---|---|---|---|
| Composition 1 | 0.90 | 9.00 | 90.10 |
| Composition 2 | 0.24 | 4.80 | 94.96 |
| Composition 3 | 3.23 | 16.13 | 80.64 |
| Composition 4 | 1.64 | 16.39 | 81.97 |
| Composition 5 | 0.83 | 16.53 | 82.64 |
| Composition 6 | 2.04 | 16.33 | 81.63 |

### Example 1.2: Compositions according to the invention

| **Substance** | **Dihydroavenanthr amide D [wt.-%]** | **Polyoxyethylene (9) tridecyl ether [wt.-%]** | **Oil bodies [wt.-%]** |
|---|---|---|---|
| Composition 7 | 0.90 | 9.00 | 90.10 |
| Composition 8 | 0.24 | 4.80 | 94.96 |
| Composition 9 | 3.23 | 16.13 | 80.64 |

### Example 1.3: Compositions according to the invention

| **Substance** | **Dihydroavenanthr amide D [wt.-%]** | **Polyoxyethylene lauryl ether [wt.-%]** | **Oil bodies [wt.-%]** |
|---|---|---|---|
| Composition 10 | 0.90 | 9.00 | 90.10 |
| Composition 11 | 0.24 | 4.80 | 94.96 |
| Composition 12 | 3.23 | 16.13 | 80.64 |

### Example 1.4: Formulation examples 1 to 14

Formulations (preparations) comprising compositions according to the invention having skin condition improving action:
- **1:**: Skin lightening day cream o/w
- **2:**: Every day shampoo
- **3:**: After sun balm
- **4:**: Deodorizing body spray
- **5:**: Sunscreen lotion (o/w, broadband protection)
- **6:**: w/o night cream
- **7:**: Anti dandruff shampoo
- **8:**: Anti- aging & barrier repair cream
- **9:**: Antiperspirant/deodorant roll-on
- **10:**: Refatting Liquid Soap
- **11:**: Refreshing hair conditioning spray
- **12:**: Shaving Cream o/w
- **13:**: Hair conditioner with UV-B/UV-A protection, rinse off
- **14:**: Hair conditioner, leave on

| **RAW MATERIAL** | **INCI** | **% BY WEIGHT/ FORMULATION EXAMPLE** | | | | | | | | | | | | | |
|---|---|---|---|---|---|---|---|---|---|---|---|---|---|---|---|
| | | **1** | **2** | **3** | **4** | **6** | **6** | **7** | **8** | **9** | **10** | **11** | **12** | **13** | **14** |
| PEG-5 Isononanoate/ | | 0,6 | 0,6 | | 1,0 | | 1,2 | | | | 1,8 | | 0,1 | | 3,5 |
| PEG-9 Tridecylether | | 1,2 | 1,2 | 1,2 | 1,0 | 1,0 | 0,6 | 0,6 | | 0,1 | 0,2 | 0,2 | 1,9 | 1,9 | |
| PEG-5 Ethylhexanoate | | | 0,6 | 0,6 | | 1.0 | | 1,2 | | 1,9 | | 1,8 | | 0,1 | |
| Dihydro avenanthramide A | | | 0,1 | | | | | | | | 0,1 | | | | |
| Dihydro avenanthramide B | | | 0,05 | | | | | | | | | | | | |
| Dihydro avenanthramide C | | | | | | | | | | 0,05 | | | | | |
| Dihydro avenanthramide D | | 0,1 | | 0,1 | 0,1 | 0,1 | 0,1 | 0,05 | 0,05 | 0,05 | | 0,2 | 0,2 | 0,02 | 0,1 |
| Avenanthramide A | | | | | 0,1 | | | | | | | | | | |
| Avenanthramide B | | | | | | | | | 0,05 | | | | | | |
| Avenanthramide C | | | | | | | | | | 0,05 | | | | | |
| Avenanthramide D | | | | | | | | | | | 0,1 | | | | |
| Abil 350 | Dimethicone | 0.5 | | 1.0 | | | | | 0.5 | | | | | 0.1 | |
| Allantoin | Allantoin | | | 0.1 | | | | | 0.25 | | | | | | |
| Aloe Vera Gel Concentrate 10/1 * | Water (Aqua). Aloe Barbadensis Leaf Juice | | | 3.0 | | | 3.0 | 0,5 | | | | | | | |
| Alugel 34 TH | Aluminium Stearate | | | | | | 1.0 | | | | | | | | |
| Antil SPA 80 | Isostearamide MIPA, Glceryl Laurate | | | | | | | | | | 1,0 | | | | |
| Butylene Glycol | Butylene Glycol | | | 5.0 | | | | | | | | | | | |
| Carbopol Ultrez-10 | Carbomer | | | | | 0.2 | | | | | | | | | |
| Ceramide BIO* | Cetylhydroxyproline Palmitamide | | | | | | | 0.2 | 0.5 | | | | | | |
| Cetiol OE | Dicaprylyl Ether | | | 4.0 | | | | | | | | | | | |
| Cetiol SB 45 | Butyrospermum Parkii (Shea Butter) | | | 1.0 | | | | | | | | | | | |
| Citric Acid 10% sol. | Citric Acid | | 1,5 | | | | | 0.3 | | | 0.5 | | | | |
| Comperlan 100 | Cocamide MEA | | | | | | | 0.5 | | | | | | | |
| Crinipan AD | Climbazole | | | | | | | 0.5 | | | | | | | |

| **RAW MATERIAL** | **INCI** | **% BY WEIGHT/ FORMULATION EXAMPLE** | | | | | | | | | | | | | |
|---|---|---|---|---|---|---|---|---|---|---|---|---|---|---|---|
| | | **1** | **2** | **3** | **4** | **6** | **6** | **7** | **8** | **9** | **10** | **11** | **12** | **13** | **14** |
| Dehyquart A CA | Cetrimonium Chloride | | | | | | | | | | | 0,2 | | 0.2 | 0.5 |
| Dehyquart SP | Quaternium-52 | | | | | | | | | | | | | 0.5 | 4.0 |
| Dow Corning 246 Fluid | Cyclohexasilox-ane and Cyclopentasilox-ane | | | | | 2.0 | | | | | | | | | |
| Dow Corning 345 Fluid | Cyclomethicone | | | | 0.5 | | | | | | | | | | |
| D-Panthenol | Panthenol | | | 1.0 | | | | | | | | | 0,5 | | |
| Dracorin^{®} CE* | Glyceryl Stearate Citrate | 5.0 | | | | | | | 1.5 | | | | | 1.0 | 1.0 |
| Dracorin^{®} GOC* | Glyceryl Oleate Citrate. Caprylic/Capric Triglyceride | | | | 2.0 | | | | | | | | | | |
| Drago-Calm* | Water, Glycerin, Avena Sativa (Oat) Kernel Extract | 0.3 | | | | | | | | | | | | | 2,0 |
| Dragoderm^{®}* | Glycerin. Triticum Vulgare (Wheat) Gluten. Water (Aqua) | | | | | | | 2.0 | | | | | | | |
| Dragosan W/O P* | Sorbitan Isostearate. Hydrogenated Castor Oil. Ceresin. Beeswax (Cera Alba) | | | | | | 6.0 | | | | | | | | |
| Dragosantol^{®} 100* | Bisabolol | 0.3 | | | 0.1 | 0.3 | 0.2 | | | 0.1 | 0.1 | | 0,3 | | |
| Dragosine^{®}* | Carnosine | | | | | | | | 0,2 | | | | | | |
| Dragoxat^{®} 89 | Ethylhexyl Isononan-oate | | | | | | | | 2.0 | | | | | | |
| Edenor K12-18 | Coconut Palmkernel Oil Fatty Acid | | | | | | | | | | | | 10,0 | | |
| Edenor L2 SM | Stearic Acid, Palmitic Acid | | | | | | | | | | | | 24,0 | | |
| EDTA B | Tetrasodium EDTA | | | | | | | | | | | | 0,2 | | |
| EDETA BD | Disodium EDTA | | 0,1 | | | 0.1 | | | | | 0.1 | | | 0.1 | |
| Emulsiphos^{®} | Potassium Cetyl Phosphate. Hydrogenated Palm Glycerides | | | | | 1.5 | | | 2.0 | | | | | | |
| Ethanol 96 % | Ethanol | | | | | | | | | 30.0 | | 48,0 | | | |
| Extrapone^{®} Rosemary GW* | Glycerin. Water (Aqua). Rosmarinus officinalis (Rosemary) Leaf Extract | | | | | | | | 0.5 | | | | | | |

| **RAW MATERIAL** | **INCI** | **% BY WEIGHT/ FORMULATION EXAMPLE** | | | | | | | | | | | | | |
|---|---|---|---|---|---|---|---|---|---|---|---|---|---|---|---|
| | | **1** | **2** | **3** | **4** | **6** | **6** | **7** | **8** | **9** | **10** | **11** | **12** | **13** | **14** |
| Extrapone^{®} Witch Hazel Distillate colourless* | Propylene Glycol. Hamamelis Virginiana (Witch Hazel) Water. Water (Aqua). Hamamelis Virginiana (Witch Hazel) Extract | | | | | | 1.0 | | | | | | | | |
| Farnesol* | Farnesol | | | | | | | | | 0.5 | | | | | |
| Fragrance* | Fragrance | 0.3 | 0,5 | 0.3 | 0.2 | 0.4 | 0.4 | 0.5 | 0.3 | 1.0 | 0,2 | 0.5 | 1,5 | 0.5 | 0.1 |
| Frescolat^{®} MGA* | Menthone Glycerol Acetal | 0.5 | | | | 0.3 | | | | | | | 0,5 | | |
| Frescolat^{®}ML cryst.* | Menthyl Lactate | | | 0.8 | | | | | | 0.2 | | | | | |
| Frescolat^{®}X-COOL* | Menthyl Ethylamido Oxalate | | | | | | | | | | 0,5 | | | | |
| Genapol LRO liquid | Sodium Laureth Sulfate | | | | | | | 37.0 | | | | | | | |
| Glycerin 99% | Glycerin | 3.0 | | 4.0 | | 4.7 | 2.0 | | 3.0 | | | | 2,0 | | |
| Glyceryl Stearate | Glyceryl Stearate | | | | | | | | 2.0 | | | | | | |
| Hydrolite^{®}-5 * | Pentylene Glycol | | | | 5.0 | | | | | | | 1,0 | | | |
| Hydroviton^{®} PLUS* | Water. Pentylene Glycol. Glycerin. Fructose. Urea. Citric Acid. Sodium Hydroxide. Maltose. Sodium PCA. Sodium Chloride. Sodium Lactate. Trehalose. Allantoin. Sodium hyaluronate. Glucose | | | 1.0 | | | | | 1,0 | | | 1.0 | | | |
| Isoadipate^{®}* | Diisopropyl Adipate | | | | | | | | | | 0,3 | 0,5 | | | |
| Isodragol^{®}* | Triisononanoin | | | | | | | | 3.0 | | | | | | |
| Isopropyl Palmitate | Isopropyl Palmitate | 4.0 | | | | | | | | | | | | | |
| Potassium Sorbate | Potassium Sorbate | | | | | | | | | | 0,2 | | | | |
| Karion F | Sorbitol | | | | | | 2.0 | | | | | | | | |
| Keltrol RD | Xanthan Gum | 0.2 | | | | 0.2 | | | | | | | | | |
| Lanette 16 | Cetyl Alcohol | 1.0 | | | | | | | | | | | | | |
| Lanette E | Sodium Cetearyl Sulfate | | | | | | | | | | | | | | |
| Lanette O | Cetearyl Alcohol | | | | | 1.0 | | | 2.0 | | | | | | |
| Lara Care A-200 | Galactoarabinan | | | 0.3 | | | | | | | | | | 0,5 | 1.5 |

| **RAW MATERIAL** | **INCI** | **% BY WEIGHT/ FORMULATION EXAMPLE** | | | | | | | | | | | | | |
|---|---|---|---|---|---|---|---|---|---|---|---|---|---|---|---|
| | | **1** | **2** | **3** | **4** | **6** | **6** | **7** | **8** | **9** | **10** | **11** | **12** | **13** | **14** |
| Magnesium Chloride | Magnesium Chloride | | | | | | 0.7 | | | | | | | | |
| Merquat 550 | Polyquaternium-7 | | | | | | | 0.5 | | | | | | | |
| NaOH 10% aqueous sol. | Sodium Hydroxide | | | | | | | | 0.3 | | | | | | |
| Natrosol 250 HHR | Hydroxyethyl-cellulose | | | | | | | | | 0.3 | | | | | |
| Neo Heliopan^{®} 357* | Butyl Methoxy-dibenzoyl-methane | | | | | 1.0 | | | | | | | | | |
| Neo Heliopan^{®} AP * (10 % as sodium salt) | Disodium Phenyl Dibenzimidazole Tetrasulfonate | | | | | 10 | | | | | | | | | |
| Neo Heliopan^{®} AV* | Ethylhexyl Methoxycinnamate | 5.0 | | | | 3.0 | | | | | | | | | |
| Neo Heliopan^{®} E1000* | Isoamyl p-Methoxycinnamate | | | | | 3,0 | | | | | | | | | |
| Neo Heliopan^{®} HMS* | Homosalate | 2,0 | | | | | | | | | | | | | |
| Neo Heliopan^{®} Hydro* (15 % as sodium salt) | Phenylbenz-imidazole Sulfonic Acid | | | | | 6.7 | | | | | | | | | |
| Neo Heliopan^{®} MBC * | 4-Methylbenzyl-idene Camphor | | | | | 1.5 | | | | | | | | | |
| Neo Heliopan^{®} OS* | Ethylhexyl Salicylate | | | | | 5.0 | | | | | | | | | |
| Neutral Oil | Caprylic/Capric Triglyceride | 6.0 | | | 4.0 | 2.0 | | | 10.0 | | | | 3,0 | | 1.0 |
| Paraffin Oil | Mineral Oil | | | | 4.0 | | | | | | | | | | |
| PCL Liquid 100* | Cetearyl Ethylhexoate | 3.0 | | | 7.0 | | 12.0 | | | | | | | | 0.3 |
| PCL Solid * | Stearyl Heptanoate. Stearyl Caprylate | | | | | | | | | | | | | 3.0 | |
| Pemulen TR-2 | Acrylates/C10-30 Alkyl Acrylate Crosspolymer | | | 0.3 | 0.2 | | | | | | | | | | |
| Plantacare PS10 | Sodium Laureth Sulfate, Lauryl Glycoside | | 17,0 | | | | | | | | | | | | |
| Polymer JR 400 | Polyquaternium-10 | | 0,2 | | | | | | | | | | | | 0.1 |
| Potassium Hydroxide 50% aqueous sol. | Potassium Hydroxide | | | | | | | | | | | | 11,0 | | |
| Propylene Glycol | Propylene Glycol | | | | | | | | | | | 0.8 | | | 0.8 |
| Sodium Ascorbyl Phosphate | Sodium Ascorbyl Phosphate | 2.0 | | 1.0 | | | | | | | | | | | |
| Sodium Benzoate | Sodium Benzoate | | | | | | | 0.5 | | | | | | | |

| **RAW MATERIAL** | **INCI** | **% BY WEIGHT/ FORMULATION EXAMPLE** | | | | | | | | | | | | | |
|---|---|---|---|---|---|---|---|---|---|---|---|---|---|---|---|
| | | **1** | **2** | **3** | **4** | **6** | **6** | **7** | **8** | **9** | **10** | **11** | **12** | **13** | **14** |
| Sodium Chloride | Sodium Chloride | | 0,4 | | | | | 1.0 | | | | | | | |
| Sodium Hydroxide (10% sol.) | Sodium Hydroxide | | | 0.6 | 0.4 | | | | | | | | 5,0 | | |
| Genapol LRO 28% AS | Sodium Laureth Sulfate | | | | | | | | | | 20,0 | | | | |
| Solubilizer 611674* | PEG-40 Hydrogenated Castor Oil. Trideceth-9. Water (Aqua) | | | | | | | | | 2.0 | | | | | |
| Sun Flower Oil | Helianthus Annuus (Sunflower) Seed Oil | | | | | | 5.0 | | | | | | | | |
| Sweet Almond Oil | Prunus dulcis | | | | | | 5.0 | | | | | | | | |
| SymCalmin^{®} | Pentylene Glycol. Butylene Glycol. Hydroxyphenyl Propamidobenzoic Acid | | | 1.0 | | | | | 1.0 | | | | | | |
| SymDeo^{®} B125* | 2-Methyl 5-Cyclohexylpentanol | | | | 0,5 | | | | | 0.5 | | | | | |
| SymDeo^{®} MPP* | Dimethyl Phenylbutanol | | | | 0.5 | | | | | | | | | | |
| Symdiol^{®}68* | 1.2-Hexanediol. Caprylylglycol. | | 1,0 | | | | | | | | 0.5 | | | | |
| Symdiol^{®}68T* | 1.2-Hexanediol. Caprylylglycol. Tropolone | 0.5 | | | | | | | | | | | | | |
| SymMatrix^{®}* | Maltodextrin. Rubus Fruticosus (Blackberry) Leaf Extract | | | | | 0.3 | 1.0 | | | | | | | | |
| SymMollient^{®}S * | Cetearyl Nonanoate | | | 1,0 | | | | | | | | | 1,5 | | |
| SymMollient^{®}WS * | PEG-5 Isononanoate (29%), Trideceth-9 (61 %), Water (10%) | | 2,5 | | | | | | | | 0,5 | | | | |
| SymOcide^{®}PS * | Phenoxyethanol. Decylene Glycol. 1.2 Hexanediol | | | | | | | 1.0 | | | | | | | |
| Sympatens AL 090 | Laureth-9 | | | | 0,5 | | | | | | | | | 1.0 | |
| SymRelief^{®} 100* | Bisabolol. Zingiber Officinale (Ginger) Root Extract | | | | 0.1 | | | | | | | | 0,2 | | |

| **RAW MATERIAL** | **INCI** | **% BY WEIGHT/ FORMULATION EXAMPLE** | | | | | | | | | | | | | |
|---|---|---|---|---|---|---|---|---|---|---|---|---|---|---|---|
| | | **1** | **2** | **3** | **4** | **6** | **6** | **7** | **8** | **9** | **10** | **11** | **12** | **13** | **14** |
| SymRepair^{®} 100* | Hexyldecanol. Bisabolol. Cetylhydroxyproline Palmitamide. Stearic Acid. Brassica Campestris (Rapeseed) Sterols | | | | | | | | 2.0 | | | | | | |
| SymSave^{®}H | Hydroxyyaceto-phenone | | 0,5 | | | | | | | | | | | | |
| SymSitive^{®}1609 * | Pentylene Glycol. 4-t-Butylcyclohexanol | | | 1.5 | | | | | | | 0.5 | | | | |
| SymSol^{®}PF3 * | Water. Pentylene Glycol. Sodium Lauryl Sulfoacetate. Sodium Oleoyl Sarcosinate. Sodium Chloride. Disodium Sulfoacetate. Sodium Oleate. Sodium Sulfate | | | | | | | | | | | | | | 1.5 |
| SymVital^{®} AgeRepair* | Zingiber Officinale (Ginger) Root Extract | 0.1 | | | | | 0.1 | | | | | | | | |
| SymWhite^{®} 377* | Phenylethyl Resorcinol | 0.5 | | | | | | | | | | | | | |
| Tego Betain L7 | Cocamidopropyl Betaine | | 7,0 | | | | | 6.0 | | | 7,5 | 1.0 | | | |
| Tegosoft PC 31 | Polyglyceryl 3- Caprate | | | | | | | | 0.3 | | | | | | |
| Tegosoft TN | C12-15 Alkyl Benzoate | | | 5.0 | | 5.0 | | | | | | | | | |
| Tocopherol Acetate | Tocopheryl Acetate | | | 0.5 | | 0.5 | 3.0 | | 0.3 | | | | | | |
| Marlipal-13/99 | Trideceth-9 | | | 1,0 | | | | | | 2,5 | | | | | |
| Triethanolamine. 99% | Triethanolamine | | | | | 0.5 | | | | | | | | | |
| Water. demin. | Water (Aqua) | Ad 100 | | | | | | | | | | | | | |
| Zirkonal L 450 | Aluminium Zirconium Pentachloro-hydrate (40 % aqueous solution) | | | | | | | | | 37,0 | | | | | |

### Example 2: In vivo application

Two preparations according to the invention were tested on 40 subjects (35 - 80 years) with visible dry skin. A double-blinded study was conducted for 4 weeks. The subjects successively used composition A and composition B, respectively, each of them for 2 weeks.

| **Substance** | **INCL** | **Composition A [Wt.-%]** | **Composition B [Wt.-%]** |
|---|---|---|---|
| NeoPcl ws | Trideceth-9, PEG-5 Ethylhexanoate | 1.00 | 1.00 |
| Water demin. | Water (Aqua) | 85.60 | 83.60 |
| SymCalmin | Butylene Glycol, Pentylene Glycol 1:1; 95%) Dihydroavenanthramide D (5%) | --- | 2.00 |
| Euxyl K400 | Methyldibromoglutaronitrile, Phenoxyethanol | 0.15 | 0.15 |
| PCL liquid 100 | Cetearyl Octanoate | 3.00 | 3.00 |
| Lanette O | Cetearyl alcohol | 2.00 | 2.00 |
| Pemulen TR1 | Acrylates/C10-30 alkyl Actrylate Cross polymere | 0.25 | 0.25 |
| Paraffin oil 5°E | Mineral Oil | 3.00 | 3.00 |
| Eutanol G | Octyldodecanol | 4.00 | 4.00 |
| Abil 350 | Dimethicone | 0.50 | 0.50 |
| Sodium hydroxide (10%) | Sodium hydroxide | 0.50 | 0.50 |
| Sum | | 100.00 | 100.00 |
| pH | | 6.10 | 6.10 |

The parameters scaling, excoriation and lichenification were measured for the compositions A and B before the study (baseline), and after 2 weeks of application time, respectively. The parameters were assessed by applying a 10-point ordinal scale with 0 = none up to 9 = severe. The score reduction of preparation A or B, respectively, as listed below, was assessed as the score reduction between the measurements of baseline and after 2 weeks.

| | Scaling | | | Excoriation | | | Lichenification | | |
|---|---|---|---|---|---|---|---|---|---|
| | Score | Score reducti on vs. Baseline | **Score reducti on vs. Base-line [%]** | Score | Score reducti on vs. Baseline | **Score reducti on vs. Base-line [%]** | Score | Score reducti on vs. Baseline | **Score reducti on vs. Base-line [%]** |
| Baseline | 6.35 | 0.00 | **100** | 5.73 | 0.00 | **100** | 5.53 | 0.00 | **100** |
| A | 4.92 | 1.43 | **22.5** | 4.56 | 1.17 | **20.4** | 4.41 | 1.12 | **20.3** |
| B | 3.35 | 3.00 | **47.2** | 2.95 | 2.78 | **48.5** | 3.00 | 2.53 | **45.7** |

Preparation A, comprising Trideceth-9 and PEG-5 Ethylhexanoate reduces (mean values of 40 subjects) the clinical symptoms associated with dry skin such as scaling, excoriation and lichenification, respectively, by 22.5%, 20.4% and 20.3%, respectively
Preparation B, comprising Dihydroavenanthramide D, Trideceth-9 and PEG-5 Ethylhexanoate shows reinforced reduction (mean values of 40 subjects) of the clinical symptoms associated with dry skin such as scaling, excoriation and lichenification, respectively, by 47.2%, 48.5% and 45.7%, respectively.

### Example 3: In vivo application

Three shampoo preparations were tested on 20 subjects (16 females and 4 males; average age: 48.9 years) who suffered from scaling on the scalp.

| **Substance** | **Shampoo 1 [Wt.-%]** | **Shampoo 2 [Wt.-%]** | **Shampoo 3 [Wt.-%]** |
|---|---|---|---|
| Dihydroavenanthramide D | 0.05 | 0.01 | - |
| Trideceth-9 | - | 0.45 | 0.6 |
| PEG-5 Ethylhexanoate | - | 0.23 | 0.3 |

The study was carried out in a temperature and humidity-controlled room (24 + /-2°C; 50 + 10% R.U.). The subjects carried out a 7-day wash out period during which they used a standardized wash out shampoo without actives.

Subsequently, each volunteer tested shampoo 1, 2 or 3, according to a randomization chart. The products were filled in anonymous containers that did not provide any information about the treatment.

At the baseline, the volunteers were clinically examined for their degree of scaling (T₀). After the basal evaluations the shampoo (active or placebo) was given to the volunteers on the basis of the randomization chart. Each volunteer used the assigned shampoo three times a week.

A clinical evaluation about the scaling reduction of the skin was performed at the end of the treatment (T₇).

| **Reduction of scaling** | **Shampoo 1 [Wt.-%]** | **Shampoo 2 [Wt.-%]** | **Shampoo 3 [Wt.-%]** |
|---|---|---|---|
| Score T₇ / T₀ | 1 | 0,778 | 1 |

In subjects treated with shampoo 1 or 3, no reduction of scaling could be observed. Patients treated with shampoo 2, however, showed a reduction of their scaling of 22.2%.

This was surprising, particularly, as shampoo 2 contains even less of the single substances than shampoo 1 or, respectively, shampoo 3. This finding was furthermore unexpected, as the sum of Dihydroavenanthramide D, Trideceth-9 and PEG-5 Ethylhexanoate in shampoo 2 is even less than the sum of Trideceth-9 and PEG-5 Ethylhexanoate in shampoo 3.

## Claims

1. Composition, comprising
a) one, two, three or more Avenanthramide(s) of formula (I) 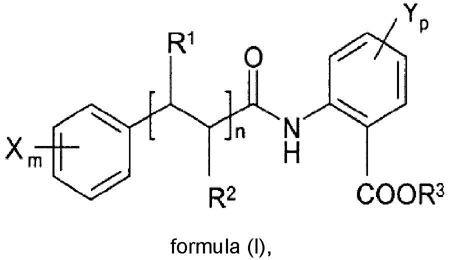 wherein
m = 0, 1,2 or 3,
p = 0, 1 or 2,
n = 0, 1 or 2,
providing that if n is 1 or 2, the sum (p + m) is > 0,
wherein if n is 1 or 2, both R¹ and R² are H or together form a further chemical bond,
wherein if m is 1, 2 or 3, each X is independent of the other(s) OH, O-Alkyl or O-Acyl,
wherein if p is 1 or 2, each Y is independent of the other(s) OH, O-Alkyl or O-Acyl,
providing that if (p + m) > 0, X or Y is at least once selected from the group consisting of OH and O-Acyl,
wherein R³ = H, N or Alkyl,
wherein the or an Avenanthramide of formula (I) is Dihydroavenanthramide D,
and
two, three or more polyalkylene glycol derivatives according to formula (II): R¹(OCH₂CHR²)ₙOR³,
in which
R¹ stands for hydrogen or a linear or branched alkyl group with 1 to 4 carbon atoms,
R² stands for hydrogen or methyl,
n represents an integer of from 3 to 20
R³ stands for a linear or branched alkyl or alkenyl group with 6 to 18 carbon atoms and 0, 1, 2 or 3 double bonds or a -COR⁴ acyl group, and
R⁴ stands for hydrogen, a linear or branched alkyl or alkenyl group having 5 to 17 carbon atoms, or an alkali metal, an alkaline earth metal or NH₃,
wherein the or two polyalkylene glycol derivatives according to formula (II) are Polyoxyethylene (9) tridecyl ether and PEG-5 Ethylhexanoate
and optionally
b) oil bodies,
wherein
the ratio of the total amount of the Avenanthramide(s) to the total amount of the polyalkylene glycol derivative(s) in the composition is in the range of from 1:25 to 1:100 based on weight.

2. Composition according to claim 1, wherein component a) consists of Dihydroavenanthramide D and Polyoxyethylene (9) tridecyl ether and PEG-5 Ethylhexanoate.

3. Composition according to any of the preceding claims, wherein the total amount of component a) and / or b), if present, in the composition is sufficient to improve or prevent one or more undesired skin condition(s), wherein the undesired skin condition(s) is / are preferably one, two or more condition(s) selected from the group consisting of excoriation, lichenification and scaling associated with dry skin.

4. Composition according to any one of the preceding claims for use in a therapeutic method for improving or preventing one or more undesired skin condition(s), wherein the undesired skin condition(s) is / are preferably one, two or more condition(s) selected from the group consisting of excoriation, lichenification and scaling associated with dry skin.

5. Cosmetic and / or dermatological preparation comprising a composition according to any one of claims 1 to 3, wherein such composition is present in a total amount sufficient to improve or prevent one or more undesired skin condition(s), wherein the undesired skin condition(s) is / are preferably one, two or more condition(s) selected from the group consisting of excoriation, lichenification and scaling associated with dry skin.

6. Cosmetic and / or dermatological preparation according to claim 5, wherein the preparation is an oil-in-water (o/w) emulsion, preferably a two-phase system of an oil in water or a shampoo.

7. Cosmetic and / or dermatological preparation according to claim 5 or claim 6, wherein the total amount of polyalkylene glycol derivative(s) accounts for 0.1 to 5.0 wt.-%, preferably 0.5 to 2.5 wt.-%, of the preparation.

8. Cosmetic and / or dermatological preparation according to any one of claims 5 to 7, wherein the total amount of the Avenanthramide(s) accounts for 0.005 to 1.0 wt.-%, preferably 0.01 to 1.0 wt.-%, preferably 0.05 to 1.0 wt.-%, preferably 0.005 to 0.20 wt.-%, preferably 0.01 to 0.20 wt.-%, 0.02 to 0.20 wt.-%, of the preparation.

9. Cosmetic and / or dermatological preparation according to any one of claims 5 to 8, wherein the preparation further comprises one or more moderately water-soluble active ingredients selected from the group consisting of odoriferous substances, perfume oils, aroma substances, aromas, fatty oils, fatty acids, waxes, ceramides, pseudoceramides, sterols, phytosterols, antiacne active ingredients, antidandruff active ingredients, antimicrobial active ingredients, preservatives, antiperspirants, antiirritants, pruritus-relieving active ingredients, cooling active ingredients, antioxidants, UV filters, antiaging active ingredients, skin-lightening and skin-tanning active ingredients, skin moisture regulators, osmolytes, insect repellents, enzyme inhibitors, odor absorbers, dyes.

10. Cosmetic and / or dermatological preparation according to any one of claims 5 to 9 for use in a therapeutic method for improving or preventing one or more undesired skin conditions, wherein the undesired skin condition(s) is / are preferably one, two or more conditions selected from the group consisting of excoriation, lichenification and scaling associated with dry skin.

## Patentansprüche

1. Zusammensetzung umfassend
a) ein, zwei, drei oder mehr Avenanthramid(e) gemäß Formel (I) 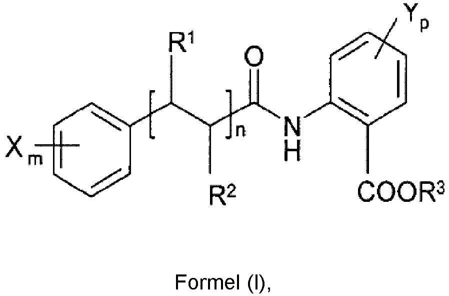 wobei
m = 0, 1, 2 oder 3,
p = 0, 1 oder 2,
n = 0, 1 oder 2,
vorausgesetzt, dass falls n gleich 1 oder 2 ist, die Summe (p + m) > 0 ist,
wobei, falls n gleich 1 oder 2 ist, R¹ und R² H sind oder zusammen eine weitere chemische Bindung bilden,
wobei, falls m gleich 1, 2 oder 3 ist, jedes X unabhängig von dem/den anderen OH, O-Alkyl oder O-Azyl ist,
wobei, falls p gleich 1 oder 2 ist, jedes Y unabhängig von dem/den anderen OH, O-Alkyl oder O-Azyl ist
vorausgesetzt dass, falls (p + m) > 0 ist, X oder Y mindestens einmal aus der Gruppe bestehend aus OH und O-Azyl ausgewählt ist,
wobei R³ = H, N oder Alkyl,
wobei das oder ein Avenanthramid gemäß Formel (I) Dihydroavenanthramid D ist
und
zwei, drei oder mehr Polyalkylenglykolderivate gemäß Formel (II):
R¹(OCH₂CHR²)ₙOR³,
worin
R¹ für Wasserstoff oder eine lineare oder verzweigte Alkylgruppe mit 1 bis 4 Kohlenstoffatomen steht,
R² für Wasserstoff oder Methyl steht,
n eine ganze Zahl von 3 bis 20 ist,
R³ für eine lineare oder verzweigte Alkyl- oder Alkenylgruppe mit 6 bis 18 Kohlenstoffatomen und 0, 1, 2 oder 3 Doppelbindungen oder einer -COR⁴ Azylgruppe steht und
R⁴ für Waserstoff, eine lineare oder verzweigte Alkyl- oder Alkenylgruppe mit 5 bis 17 Kohlenstoffatomen oder ein Alkalimetal, ein Erdalkalimetall oder NH₃ steht
wobei die oder zwei Polyalkylenglykolderivate gemäß Formel (II) Polyoxyethylen(9)tridezylether und PEG-5-Ethylhexanoat sind
und optional
b) Ölkörper,
wobei das Verhältnis der Gesamtmenge des/der Avenanthramid(e) zur Gesamtmenge der Polyalkylenglykolderivate in der Zusammensetzung in einem Bereich von 1:25 bis 1:100, basierend auf dem Gewicht, liegt.

2. Zusammensetzung nach Anspruch 1, wobei Komponente a) aus Dihydroavenanthramid D und Polyoxyethylen(9)tridezylether und PEG-5-Ethylhexanoat besteht.

3. Zusammensetzung nach einem der vorangehenden Ansprüche, wobei die Gesamtmenge an Komponente a) und/oder b), sofern vorhanden, in der Zusammensetzung ausreicht, um einen oder mehrere unerwünschte(n) Hautzustand/Hautzustände zu verbessern oder verhindern, wobei der/die unerwünschte(n) Hautzustand/Hautzustände vorzugsweise ein, zwei oder mehrere Zustände ausgewählt aus der Gruppe bestehend aus Exkoriation, Lichenifikation und Hautabschuppung, die mit trockener Haut in Verbindung gebracht wird, ist/sind.

4. Zusammensetzung nach einem der vorangehenden Ansprüche zur Anwendung in einem therapeutischen Verfahren zum Verbessern oder Vorbeugen eines oder mehrerer unerwünschter Hautzustände, wobei der/die unerwünschte(n) Hautzustand/Hautzustände vorzugsweise ein, zwei oder mehrere Zustände ausgewählt aus der Gruppe bestehend aus Exkoriation, Lichenifikation und Hautabschuppung, die mit trockener Haut in Verbindung gebracht wird, ist/sind.

5. Kosmetische und/oder dermatologische Zubereitung umfassend eine Zusammensetzung nach einem der Ansprüche 1 bis 3, wobei eine solche Zusammensetzung in einer Gesamtmenge vorhanden ist, die ausreicht, um einen oder mehrere unerwünschte(n) Hautzustand/Hautzustände zu verbessern oder verhindern, wobei der/die unerwünschte(n) Hautzustand/Hautzustände vorzugsweise ein, zwei oder mehrere Zustände ausgewählt aus der Gruppe bestehend aus Exkoriation, Lichenifikation und Hautabschuppung, die mit trockener Haut in Verbindung gebracht wird, ist/sind.

6. Kosmetische und/oder dermatologische Zubereitung nach Anspruch 5, wobei die Zubereitung eine Öl-in-Wasser (o/w) Emulsion, vorzugsweise ein Zwei-Phasen-System einer Öl-in-Wasser Emulsion oder ein Shampoo ist.

7. Kosmetische und/oder dermatologische Zubereitung nach Anspruch 5 oder 6, wobei die Gesamtmenge an Polyalkylenglykolderivat(en) 0,1 bis 5 Gew.-%, vorzugsweise 0,5 bis 2,5 Gew.-% der Zubereitung ausmacht.

8. Kosmetische und/oder dermatologische Zubereitung nach einem der Ansprüche 5 bis 7, wobei die Gesamtmenge des/der Avenanthramid(e) 0,005 bis 1,0 Gew.-%, vorzugsweise 0,01 bis 1,0 Gew.-%, vorzugsweise 0,05 bis 1,0 Gew.-%, vorzugsweise 0,005 bis 0,20 Gew.-%, vorzugsweise 0,01 bis 0,20 Gew.-%, 0,02 bis 0,20 Gew.-% der Zubereitung ausmacht.

9. Kosmetische und/oder dermatologische Zubereitung nach einem der Ansprüche 5 bis 8, wobei die Zubereitung zudem einen oder mehrere moderat wasserlösliche Wirkstoffe enthält, ausgewählt aus der Gruppe bestehend aus duftenden Substanzen, Parfümölen, Aromastoffen, Aromen, Fettölen, Fettsäuren, Wachsen, Ceramiden, Pseudoceramiden, Sterolen, Phytosterolen, anti-Akne-Wirkstoffen, Antischuppenwirkstoffen, antimikrobiellen Wirkstoffen, Konservierungsmitteln, Antiperspirantien, Antiirritantien, Juckreiz lindernden Wirkstoffen, Kühlwirkstoffen, Antioxidantien, UV-Filtern, anti-aging-Wirkstoffen, Haut aufhellenden und Haut bräunenden Wirkstoffen, Hautfeuchtigkeitsregulatoren, Osmolyten, Insektenschutzmitteln, Enzyminhibitoren, Geruchsabsorbern, Farbstoffen.

10. Kosmetische und/oder dermatologische Zubereitung nach einem der Ansprüche 5 bis 9 zur Anwendung in einem therapeutischen Verfahren zum Verbessern oder Vorbeugen eines oder mehrerer unerwünschter Hautzustände, wobei der/die unerwünschte(n) Hautzustand/Hautzustände vorzugsweise ein, zwei oder mehrere Zustände ausgewählt aus der Gruppe bestehend aus Exkoriation, Lichenifikation und Hautabschuppung, die mit trockener Haut in Verbindung gebracht wird, ist/sind.

## Revendications

1. Composition comprenant
a) un, deux, trois avenanthramide(s) ou plus, de formule (I) 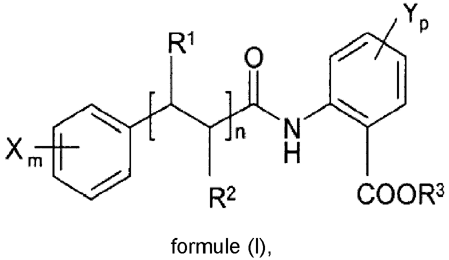 dans laquelle
m = 0, 1, 2 ou 3,
p = 0, 1 ou 2,
n = 0, 1 ou 2,
à condition que, si n est 1 ou 2, la somme (p + m) soit > 0,
dans laquelle, si n est 1 ou 2, R¹ et R² sont tous deux H ou forment ensemble une autre liaison chimique,
dans laquelle, si m est 1, 2 ou 3, chaque X est, indépendamment de l'autre/des autres OH, O-alkyle ou O-acyle,
dans laquelle, si p est 1 ou 2, chaque Y est, indépendamment de l'autre/des autres OH, O-alkyle ou O-acyle,
à condition que, si (p + m) > 0, X ou Y soit au moins une fois choisi dans le groupe constitué par OH et O-acyle,
dans laquelle R³ = H, N ou alkyle,
dans laquelle le ou un avenanthramide de formule (I) est le dihydroavenanthramide D,
et
deux, trois dérivés de polyalkylène glycol ou plus, selon la formule (II):
R¹(OCH₂CHR²)ₙOR³,
dans laquelle
R¹ représente l'hydrogène ou un groupe alkyle linéaire ou ramifié ayant 1 à 4 atomes de carbone,
R² représente l'hydrogène ou le méthyle,
n représente un nombre entier de 3 à 20
R³ représente un groupe alkyle ou alcényle linéaire ou ramifié ayant 6 à 18 atomes de carbone et 0, 1, 2 ou 3 doubles liaisons ou un groupe acyle-COR⁴, et
R⁴ représente l'hydrogène, un groupe alkyle ou alcényle linéaire ou ramifié ayant 5 à 17 atomes de carbone ou un métal alcalin, un métal alcalino-terreux ou NH₃,
dans laquelle le ou deux dérivés de polyalkylène glycol selon la formule (II) sont l'éther tridécylique de polyoxyéthylène (9) et PEG-5 éthylhexanoate
et en option
b) des corps huileux,
dans laquelle
le rapport de la quantité totale de l'/des avenanthramide(s) à la quantité totale des dérivé(s) de polyalkylène glycol dans la composition est dans la plage allant de 1 : 25 à 1 : 100, sur la base du poids.

2. Composition selon la revendication 1, dans laquelle le composant a) est constitué de dihydroavenanthramide D et d'éther tridécylique de polyoxyéthylène (9) et de PEG-5 éthylhexanoate.

3. Composition selon l'une quelconque des revendications précédentes, dans laquelle la quantité totale du composant a) et/ou b), s'il est présent, dans la composition est suffisante pour améliorer ou prévenir une ou plusieurs condition(s) cutanée(s) indésirable(s), dans laquelle la / les condition(s) cutanée(s) indésirable(s) est / sont de préférence une, deux ou plusieurs condition(s) choisie(s) dans le groupe constitué par l'excoriation, la lichénification et la desquamation qui sont associées à la peau sèche.

4. Composition selon l'une quelconque des revendications précédentes, destinée à être utilisée dans une méthode thérapeutique pour améliorer ou prévenir une ou plusieurs condition(s) cutanée(s) indésirable(s), dans laquelle la / les condition(s) cutanée(s) indésirable(s) est / sont de préférence une, deux ou plusieurs condition(s) choisie(s) dans le groupe constitué par l'excoriation, la lichénification et la desquamation qui sont associées à la peau sèche.

5. Préparation cosmétique et/ou dermatologique comprenant une composition selon l'une quelconque des revendications 1 à 3, dans laquelle une telle composition est présente en une quantité totale suffisante pour améliorer ou prévenir une ou plusieurs condition(s) cutanée(s) indésirable(s), dans laquelle la / les condition(s) cutanée(s) indésirable(s) est / sont de préférence une, deux ou plusieurs condition(s) choisie(s) dans le groupe constitué par l'excoriation, la lichénification et la desquamation qui sont associées à la peau sèche.

6. Préparation cosmétique et/ou dermatologique selon la revendication 5, dans laquelle la préparation est une émulsion huile dans eau (o/w), de préférence un système biphasique d'une émulsion huile dans eau ou un shampooing.

7. Préparation cosmétique et/ou dermatologique selon la revendication 5 ou la revendication 6, dans laquelle la quantité totale de dérivé(s) de polyalkylène glycol représente 0,1 à 5,0 % en poids, de préférence 0,5 à 2,5 % en poids, de la préparation.

8. Préparation cosmétique et/ou dermatologique selon l'une quelconque des revendications 5 à 7, dans laquelle la quantité totale de l'/des avenanthramide(s) représente 0,005 à 1,0 % en poids, de préférence 0,01 à 1,0 % en poids, de préférence 0,05 à 1,0 % en poids, de préférence 0,005 à 0,20 % en poids, de préférence 0,01 à 0,20 % en poids, 0,02 à 0,20 % en poids de la préparation.

9. Préparation cosmétique et/ou dermatologique selon l'une quelconque des revendications 5 à 8, dans laquelle la préparation comprend en outre un ou plusieurs principes actifs modérément hydrosolubles qui sont choisis dans le groupe constitué par les substances odorantes, les huiles parfumées, les substances aromatiques, les arômes, les huiles grasses, les acides gras, les cires, les céramides, les pseudo-céramides, les stérols, les phytostérols, les principes actifs antiacnéiques, les principes actifs antipelliculaires, les principes actifs antimicrobiens, les conservateurs, les antitranspirants, les antiirritants, les principes actifs antiprurigineux, les principes actifs rafraîchissants, les antioxydants, les filtres UV, les principes actifs anti-âge, les principes actifs éclaircissants et bronzants, les régulateurs d'hydratation cutanée, les osmolytes, les insectifuges, les inhibiteurs d'enzymes, les absorbeurs d'odeurs, les colorants.

10. Préparation cosmétique et/ou dermatologique selon l'une quelconque des revendications 5 à 9 , destinée à être utilisée dans une méthode thérapeutique pour améliorer ou prévenir une ou plusieurs condition(s) cutanée(s) indésirable(s), dans laquelle la / les condition(s) cutanée(s) indésirable(s) est / sont de préférence une, deux ou plusieurs condition(s) choisie(s) dans le groupe constitué par l'excoriation, la lichénification et la desquamation qui sont associées à la peau sèche.
